# DEMANDE DE BREVET EUROPEEN

(11) **EP 1 393 762 A1**
(43) Date de publication de la demande: **03.03.2004**
(21) Numéro de dépôt: 02405736.6
(22) Date de dépôt: 29.08.2002
(51) Int. Cl.: A61M 5/142, F04B 43/12

(54) **Pompe médicale péristaltique**

(71) Demandeur: Precimedix S.A., 2000 Neuchâtel (CH)
(72) Inventeur: Wüthrich, Heinz, 3048 Worblaufen (CH); Möri, Peter, 3272 Walperswil (CH); Stulz, Beat, 1700 Fribourg (CH); Clement, Claude, 1333 Lussy-sur-Morges (CH)
(74) Mandataire: Gresset, Jean

(57) **Abrégé**

L'invention concerne une pompe péristaltique pour l'injection de médicaments comprenant deux modules s'accouplant l'un à l'autre, soit :
- un module cassette (12) comportant, à l'intérieur d'un boîtier (30), un réservoir destiné à contenir une solution médicamenteuse, un tuyau souple relié audit réservoir et une pièce d'appui (34) sur laquelle est positionné le tuyau, et
- un module pompe (10) comportant, à l'intérieur d'un boîtier (16), une tête de pompe (20) dotée de galets presseurs (22) destinés, lorsque les deux modules sont accouplés, à écraser le tuyau pour faire avancer la solution médicamenteuse et des moyens d'entraînement en rotation de la tête.

Le module pompe (10) comporte une membrane à faible coefficient de frottement (38) tendue sur un cadre (40) disposé et conformé de manière à ce que la membrane s'interpose entre les galets (22) et la pièce d'appui (34) en épousant la partie de la tête de pompe (20) qui agit sur le tuyau.

## Description

La présente invention se rapporte au domaine des pompes miniaturisées pour l'injection de médicaments selon un programme prescrit. Elle concerne, plus particulièrement, une pompe médicale programmable formée d'un module pompe et d'un module cassette contenant le médicament, qui s'accouplent de manière amovible.

Les pompes miniaturisées à usage médical sont connues depuis plusieurs années. Légères et de faibles dimensions, elles sont portées par le patient de façon discrète et sans inconfort et permettent de lui administrer, par voie sous-cutanée ou intraveineuse, en continu ou selon un programme déterminé, des quantités contrôlées de solutions médicamenteuses, sans qu'il soit, pour autant, alité et relié à un appareil encombrant, coûteux et bruyant.

De telles pompes sont, souvent, de type péristaltique rotatif. Leur principe consiste à disposer d'un tuyau en matière plastique déformable relié à un réservoir contenant la solution de médicament et à l'écraser localement contre une pièce d'appui de forme arrondie au moyen de galets presseurs montés sur un rotor entraîné par un moteur agissant par l'intermédiaire d'un train d'engrenages. Le liquide est ainsi aspiré du réservoir et poussé vers la sortie pour être injecté dans le corps du patient.

Les pompes péristaltiques, de par leur conception, posent certains problèmes. Notamment, il arrive que les galets presseurs ne roulent pas correctement sur le tuyau, mais, au lieu de cela, aient tendance à l'agripper. Ce dernier, sous l'effet de la force de friction, peut alors quitter son emplacement et se gondoler, jusqu'à ce que la tension élastique ainsi créée lui fasse soudainement reprendre sa place initiale, ce qui perturbe considérablement le débit d'écoulement du fluide.

Par ailleurs, une pompe péristaltique à deux modules accouplables souffre du fait que ceux-ci sont nécessairement ouverts du côté où s'effectue l'accouplement. La jonction n'étant pas étanche, les organes mécaniques sont ainsi exposés aux poussières, aux infiltrations d'humidité..., ce qui peut nuire au bon fonctionnement de la pompe.

Certes, le brevet US 4 576 556 propose, notamment pour remédier au problème de glissement des galets, d'entourer la tête de pompe d'une bague réalisée en un matériau possédant un coefficient de frottement élevé et s'interposant ainsi entre le tuyau et les galets qui peuvent alors rouler correctement et écraser le tuyau sans le pousser. La solution proposée ne permet pas, toutefois, de protéger les organes du module pompe contre les agressions extérieures.

La présente invention a pour but de pallier simultanément les deux inconvénients évoqués ci-dessus, non seulement en empêchant les galets d'entraîner le tuyau, mais encore, en préservant les organes mécaniques de la pompe des perturbations occasionnées par le milieu extérieur.

De façon plus précise, l'invention concerne une pompe péristaltique pour l'injection de médicaments, du type comprenant deux modules s'accouplant l'un à l'autre, soit :
- un module cassette comportant, à l'intérieur d'un boîtier, un réservoir destiné à contenir une solution médicamenteuse, un tuyau souple relié audit réservoir et une pièce d'appui sur laquelle est positionné ledit tuyau, et
- un module pompe comportant, à l'intérieur d'un boîtier, une tête de pompe dotée de galets presseurs destinés, lorsque les deux modules sont accouplés, à écraser ledit tuyau pour faire avancer la solution médicamenteuse et des moyens d'entraînement en rotation de ladite tête.

La pompe telle que définie ci-dessus est caractérisée en ce que le module pompe comporte une membrane à faible coefficient de frottement montée tendue sur un cadre disposé et conformé de manière à ce que ladite membrane s'interpose entre les galets et la pièce d'appui en épousant la partie de la tête de pompe qui agit sur le tuyau.

De façon avantageuse, le cadre est fixé de manière étanche au boîtier du module pompe et conformé pour constituer, avec sa membrane, une cloison de protection de son contenu.

Selon un mode de réalisation préféré, le module cassette comporte une carte mémoire contenant des données pour la commande des moyens d'entraînement du module pompe et le module pompe comporte un connecteur relié aux moyens d'entraînement et positionné de manière à ce que, lors de l'accouplement des deux modules, ses plages de connexion viennent précisément au contact de celles de la carte mémoire. Dans ce cas, le cadre est conformé pour autoriser le contact entre les plages de connexion des deux modules.

Les caractéristiques de l'invention seront mieux comprises à la lumière de la description qui suit, faite en référence au dessin annexé, dans lequel :

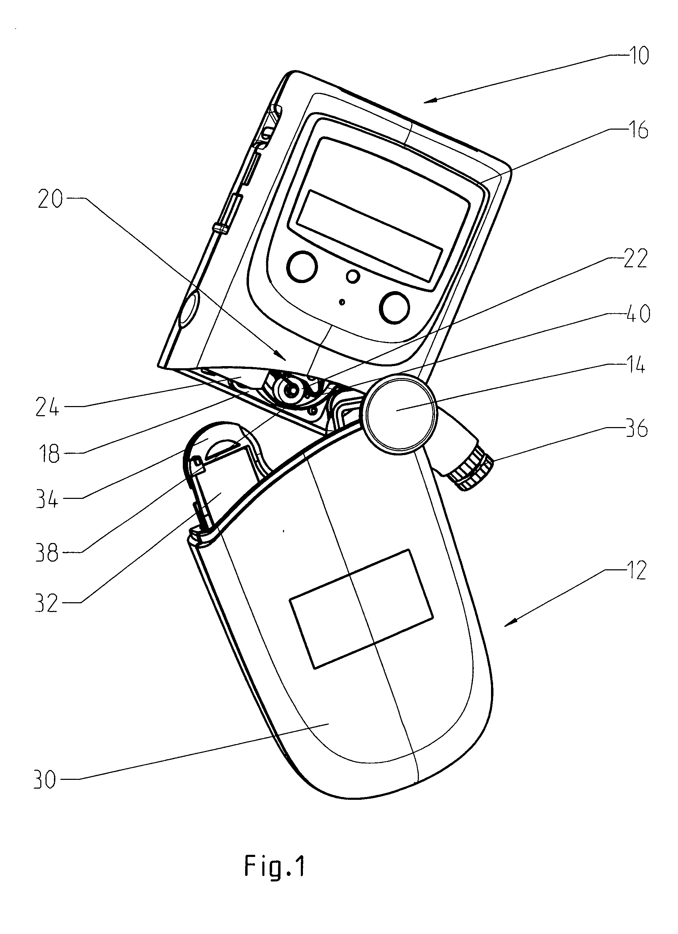

- la figure 1 est une vue d'ensemble de la pompe selon l'invention, à l'état ouvert ; et
- la figure 2 représente le module pompe, sans son circuit imprimé, la membrane et son cadre étant montrés séparément en 2a et installés en 2b, où son couvercle est éliminé.

Les figures montrent une pompe pour l'administration de médicaments, formée d'un module pompe 10 et d'un module cassette 12 qui s'accouplent l'un à l'autre de manière amovible au moyen d'une articulation à baïonnette 14. Cette pompe est celle qui fait l'objet de la demande de brevet EP 02 405659.2. Elle ne sera donc pas décrite en détail.

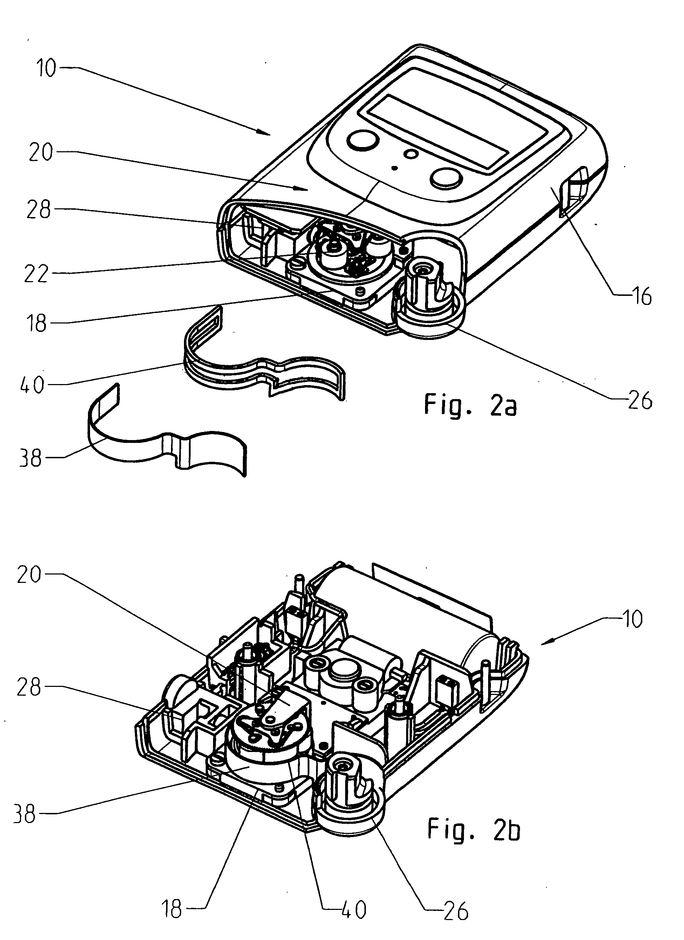

Le module pompe 10 laisse apparaître, du côté ouvert de son boîtier 16 :
- une platine métallique 18 qui sert de base à une tête de pompe 20 péristaltique rotative à trois galets presseurs 22, entraînée en rotation par un moteur (non visible au dessin);
- l'extrémité d'un circuit imprimé 24, éliminé de la figure 2, qui est doté, sur sa face interne, d'un connecteur (non visible au dessin) pour assurer la liaison électrique du module avec l'extérieur et servant de support d'interconnexion à un microprocesseur (non visible au dessin) pour la commande du moteur ;
- la partie mâle 26 de l'articulation à baïonnette 14 (visible seulement sur la figure 2); et
- la première partie 28 d'un verrou (visible seulement sur la figure 2) solidarisant les deux modules.

Le module cassette 12 laisse apparaître, du côté ouvert de son boîtier 30, une carte mémoire 32, de type SIM (Subscriber Information Memory) contenant des données relatives au programme d'injection prescrit. Cette carte est positionnée de manière à ce que, lors de l'accouplement des deux modules, ses plages de connexion viennent précisément au contact de celles du connecteur appartenant au module pompe 10.

Comme décrit dans le document EP déjà cité, la carte 32 est fixée sur une contre-pièce 34 qui porte également la partie femelle de l'articulation à baïonnette 14 et la deuxième partie du verrou. Le module cassette 12 renferme une poche réservoir de médicament qu'un tuyau souple relie à un raccord 36 destiné à connecter une tubulure se terminant par une aiguille d'injection pour administrer le médicament au patient. La contre-pièce 34 comporte, en son milieu, une partie concave arrondie sur laquelle est disposé le tuyau souple et définissant une zone d'appui contre laquelle, une fois les deux modules assemblés, lors de la rotation de la tête de pompe 20, les trois galets presseurs 22 viennent tour à tour écraser le tuyau et pousser ainsi vers l'extérieur, par un mouvement péristaltique, le liquide contenu dans la poche réservoir.

Les figures montrent en 38 une membrane à faible coefficient de frottement, typiquement réalisée en matière plastique souple mais peu extensible (PTFE, PET, Mylar, Kapton ou produit similaire), qui est montée tendue sur un cadre rigide 40 disposé à l'entrée du boîtier 16 du module pompe 10. Ce cadre est conformé de manière à ce que la membrane 38 s'interpose entre les galets 22 et la zone d'appui de la contre-pièce 34 en épousant la partie de la tête de pompe 20 qui agit sur le tuyau souple.

Ainsi, du fait du faible coefficient de frottement du matériau constituant la membrane 38, les galets 22 n'agrippent pas le tuyau mais roulent sur lui en l'écrasant de manière à éviter les à-coups et assurer une action péristaltique correcte.

Par ailleurs, de part et d'autre de la tête 20, le cadre 40 pénètre dans le boîtier 16 de manière à laisser accessibles de l'extérieur la partie mâle 26 de l'articulation à baïonnette, la partie du verrou 28 et l'extrémité du circuit imprimé 24 portant le connecteur.

Le cadre 40 est fixé au boîtier 16 de manière étanche, par collage, thermo-soudage ou toute autre technique adaptée. Il constitue ainsi, en plus, avec sa membrane 38, qui évite déjà le grippage des galets 22 et les à-coups résultants, une cloison de protection des composants du module pompe 10 contre les poussières et l'humidité, tout en autorisant la liaison mécanique et électrique avec le module cassette 12.

La fiabilité de la pompe est ainsi fortement améliorée et le patient peut l'utiliser sans avoir à prendre de précautions particulières quant à son environnement.

## Revendications

1. Pompe péristaltique pour l'injection de médicaments comprenant deux modules s'accouplant l'un à l'autre, soit :
- un module cassette (12) comportant, à l'intérieur d'un boîtier (30), un réservoir destiné à contenir une solution médicamenteuse, un tuyau souple relié audit réservoir et une pièce d'appui (34) sur laquelle est positionné ledit tuyau, et
- un module pompe (10) comportant, à l'intérieur d'un boîtier (16), une tête de pompe (20) dotée de galets presseurs (22) destinés, lorsque les deux modules sont accouplés, à écraser ledit tuyau pour faire avancer la solution médicamenteuse et des moyens d'entraînement en rotation de ladite tête,
**caractérisée en ce que** le module pompe (10) comporte une membrane à faible coefficient de frottement (38) tendue sur un cadre (40) disposé et conformé de manière à ce que ladite membrane s'interpose entre les galets (22) et la pièce d'appui (34) en épousant la partie de la tête de pompe (20) qui agit sur le tuyau.

2. Pompe selon la revendication 1, **caractérisée en ce que** le cadre (40) est fixé de manière étanche au boîtier (16) du module pompe et conformé pour constituer, avec sa membrane (38), une cloison de protection de son contenu.

3. Pompe selon l'une des revendications 1 et 2, dans laquelle le module cassette (12) comporte une carte mémoire (32) contenant des données pour la commande des moyens d'entraînement du module pompe (10) et ledit module pompe comporte un connecteur relié auxdits moyens d'entraînement et positionné de manière à ce que, lors de l'accouplement des deux modules, ses plages de connexion viennent précisément au contact de celles de ladite carte mémoire (34), **caractérisée en ce que** le cadre (40) est conformé pour autoriser le contact entre lesdites plages.

4. Pompe selon l'une des revendications 1 à 3, dans laquelle les deux modules s'accouplent au moyen d'une articulation à baïonnette (14) dont les parties mâle et femelle sont respectivement solidaires desdits boîtiers, **caractérisée en ce que** le cadre (40) est conformé pour autoriser l'accouplement desdites parties.
